# EUROPEAN PATENT SPECIFICATION

(11) **EP 3 219 306 B1**
(45) Date of publication and mention of the grant of the patent: **16.11.2022**
(21) Application number: 17161008.2
(22) Date of filing: 15.03.2017
(51) Int. Cl.: A61K 31/185, A61P 27/02, A61P 27/06, A61P 25/28, A23L 33/10, A61K 31/7068, A61K 31/355, A61K 31/353, A61K 9/00, A61K 9/24, A61K 9/28, A23L 33/15

(54) **COMPOSITION COMPRISING CITICOLINE AND HOMOTAURINE FOR PREVENTING AND TREATING NEUODEGENERATIVE DISEASES AND COGNITIVE DISORDERS**
ZUSAMMENSETZUNG ENTHALTEND CITICOLINE UND HOMOTAURIN ZUR VORBEUGUNG UND BEHANDLUNG NEUODEGENERATIVER ERKRANKUNGEN UND KOGNITIVER STÖRUNGEN
COMPOSITION COMPRENANT DE LA CITICOLINE ET DE L'HOMOTAURINE POUR LA PRÉVENTION ET LE TRAITEMENT DE MALADIES NEURO-DÉGÉNÉRATIVES ET DE TROUBLES COGNITIFS

(30) Priority: 15.03.2016 IT UA20161679
(43) Date of publication of application: 20.09.2017
(73) Proprietor: NEURAXPHARM ITALY S.P.A., 63100 Ascoli Piceno (AP) (IT)
(72) Inventor: MARCHETTI, Marco, 63074 SAN BENEDETTO DEL TRONTO (IT); BELLINI, Francesco, Calgary, Alberta T2P 0C3 (CA); CARBONI, Paolo, 63074 SAN BENEDETTO DEL TRONTO (IT)
(74) Representative: Coppo, Alessandro

(56) References cited:
- WO-A2-2007/049098
- US-A- 6 015 835
- US-A1- 2003 114 415
- US-A1- 2010 130 537
- US-A1- 2012 316 128
- Judes Poirier ET AL: "Pharmacogenomics and the Treatment of Sporadic Alzheimers Disease:A Decade of Progress", Current Pharmacogenomics and Personalized Medicine, 1 March 2008 (2008-03-01), pages 63-76, XP055315182, DOI: 10.2174/187569208784017494 Retrieved from the Internet: URL:http://adgen.myasustor.com/index_htm_f iles/Current%20Pharmacogen%20Pers%20Med%20 6_63.pdf [retrieved on 2016-10-28]
- SERGIO DAVINELLI ET AL: "Cytoprotective Effects of Citicoline and Homotaurine against Glutamate and High Glucose Neurotoxicity in Primary Cultured Retinal Cells", OXIDATIVE MEDICINE AND CELLULAR LONGEVITY, vol. 2017, 1 January 2017 (2017-01-01), pages 1-7, XP055733693, US ISSN: 1942-0900, DOI: 10.1155/2017/2825703

## Description

### FIELD OF THE INVENTION

The present invention relates to a composition for preventing or treating neurodegenerative diseases and cognitive disorders.

The present invention originates in the field of pharmaceuticals, nutraceuticals and food supplements.

In particular, the present invention relates to a preparation for oral administration suitable for preventing or treating cognitive disorders and brain involutional processes and degenerative processes of the nerve cells as further defined in the claims.

### PRIOR ART

Cognitive disorders notoriously represent organic dysfunctions of the main intellectual functions acquired such as memory, abstract thinking, critical ability, language, spatial-temporal orientation, in which the state of consciousness is still retained.

Cognitive disorders typically affect the adult population over the age of 50-60. In particular, it was found that about 5% of the population over the age of 65 and about 30% of the population over the age of 85 suffers from a cognitive disorder.

The main cognitive disorders may be classified as pathologies of three types: reversible, vascular and degenerative diseases.

In the first type, the disease onset is traumatic or resulting from an external agent, such as infection, and tends to regress once the triggering cause is corrected.

Conversely, the other two types, more representative of cognitive disorders, have a progressive degeneration matrix. Being chronic diseases, it is not possible to restore the initial physiological condition but one can only slow the disease progression and control the related symptoms. Typical examples of vascular degenerative diseases include cerebrovascular insufficiency, stroke, transient ischemic attack and cerebral hemorrhages.

Generally, when the first symptoms of a form of brain decay occur, there are the directions to establish an adequate therapeutic treatment aimed at slowing the disease progression and preventing acute events.

The type of therapeutic treatment is selected according to the severity of symptoms and the age of the individual.

The initial forms of cognitive decline are generally treated by integrating the diet with specific nutritional products, herbal products having an antioxidant or cerebrovascular action.

The patent US 6 015 835 discloses the combination of taurine, homotaurine or methionine with several other compounds including nicholin which is another name for the compound citicoline. This patent also discloses drug combinations of taurine with other medications aimed at reducing the production of free radicals.

The International patent application WO 2007/049098 A2 discloses therapeutic formulations comprising 3-amino-1-propanesulfonic acid, also known as homotaurine for the treatment of β-amyloid-related diseases such as Alzheimer's disease.

The patent application US 2010/130537 discloses that citicoline can be used for the treatment of dementia and mild cognitive impairment and that citicoline is a cholinesterase inhibitor.

Nutraceutical products currently used to prevent or treat mild forms of cognitive decline generally contain vitamins, antioxidant substances such as catecholamines, flavonoids, or selenium, resveratrol, products containing substances with a phospholipid matrix such as phosphatidylserine, phosphatidylcholine or phosphatidylinositol, or substances that control blood lipids such as omega-3 and omega-6 acids.

Conversely, drug therapy is reserved for the treatment of medium to high gravity forms in which the symptoms linked to cognitive decline begin to affect the quality of life and the performance of daily activities of the individual.

Drugs currently most widely used for the treatment of forms of medium-high severity cognitive decline, such as senile dementia and Alzheimer's disease, comprise acetylcholinesterase inhibitors, as, for example, donepezil, rivastigmine and galantamine. Other drugs such as memantine are reserved to the treatment of the most severe forms of neurodegenerative diseases and Alzheimer's disease. However, it was found that the administration of these drugs is accompanied, in most cases, by the appearance of side effects that may become important in the case of chronic drug therapies.

In addition, the nutraceutical products currently on the market and suitable for the treatment of mild or moderate forms of cognitive disorders provide an inadequate, if not totally unsatisfactory, therapeutic response.

Currently, therefore, a vacuum is found in the market as to products for the treatment of involutional disease and mild cognitive disorders, as there are almost no available products that perform a nootropic action that is substantially devoid of side effects even in case of prolonged or chronic treatments.

It is a general object of the present invention to provide a drug or nutraceutical product suitable for improving the clinical condition and/or symptoms associated with mild to moderate forms of cognitive disorders, as further defined in the claims.

Another object of the present invention is to provide a composition for preventing or treating neurodegenerative disorders and cognitive disorders, as further defined in the claims, whose chronic administration is not accompanied by severe side effects and which allows the long term treatment of neurodegenerative disorders.

A further object of the present invention is a method for preventing or treating neurodegenerative disorders or cognitive disorders, as further defined in the claims, in a mammal, in particular a human being.

### SUMMARY OF THE INVENTION

According to a first aspect of the present invention, the inventors have found that by administering a combination of the two active ingredient citicoline and homotaurine, a synergistic action of cholinergic transmission between neurons is obtained which leads to an improvement of cognitive abilities and a slowdown in the progression of the decline of cognitive functions in the subject treated.

The effect found was surprising since homotaurine and citicoline, the two basic components of the composition for use of the invention, have different biological targets and act centrally through different mechanisms of action that do not seem to justify the synergistic nootropic action found by the authors.

Therefore, according to a first aspect thereof, a composition is provided for use as claimed in the accompanying claim 1.

Further embodiments of the composition for use of the invention are defined by claims 2-8 herein enclosed.

According to a general aspect a composition for preventing and/or treating neurodegenerative and/or cognitive disorders in a mammal, typically a human being is disclosed herein.

In accordance with a primary aspect of the present invention, there is provided a composition comprising a combination of citicoline or a pharmaceutically acceptable salt thereof and homotaurine and a physiologically acceptable carrier and/or excipient for use in the prevention or treatment of a neurodegenerative disease and/or a reversible cognitive disorder, a vascular cognitive impairment and/or a neurodegenerative ocular disease, wherein citicoline is in amount of 100 to 5000 mg and homotaurine is in amount of 5 to 500 mg.

According to certain embodiments of the invention, the inventors have also found that the synergistic action of the combination of citicoline and homotaurine is incremented by the presence of a substance provided with cellular antioxidant activity, typically vitamin E.

Typically, in the composition of the invention the citicoline and homotaurine active ingredients are provided in a pharmaceutically, nutraceutical or dietetic effective quantity.

The composition of the invention can be applied to the prevention or treatment of cognitive disorders, as further defined in the claims, irrespective of their origin and/or as an adjuvant against the symptoms associated with these disorders.

According to some embodiments, the composition of the invention is a pharmaceutical composition, a nutraceutical product, a dietary supplement that can be introduced into the diet of an individual suffering from a cognitive disorder. According to some aspects, the invention provides a preparation based on citicoline and homotaurine, optionally vitamin E and a physiologically acceptable carrier as a combined preparation for simultaneous, separate or subsequent use in the prophylaxis or treatment of neurodegenerative diseases and/or cognitive disorders, as further defined in the claims.

A composition is disclosed herein that comprises a combination of citicoline and homotaurine and a physiologically acceptable carrier and/or excipient for use in the prevention or treatment of neurodegenerative diseases and cognitive disorders, as further defined in the claims.

The present invention will be described in detail below with reference to the following figures.

### BRIEF DESCRIPTION OF THE FIGURES

Some features and advantages of the present invention will become apparent from the accompanying figures, in which:

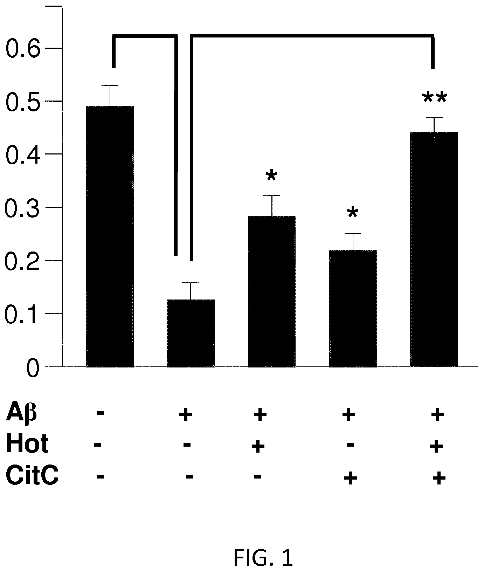

Figure 1 shows bar graphs that show the results of MMT analysis on rat hippocampal neuronal cells in order to detect the cell viability after treatment with homotaurine (Hot), citicoline (CitC) and a combination thereof against the cytotoxicity induced by Ab25-35 in rat hippocampal neuronal cells (n = 5). **, p,0.001; *, p,0.01 vs Ab25-35. The Figure shows that the citicoline and homotaurine combination have a synergistic effect of contrast to cytotoxicity.

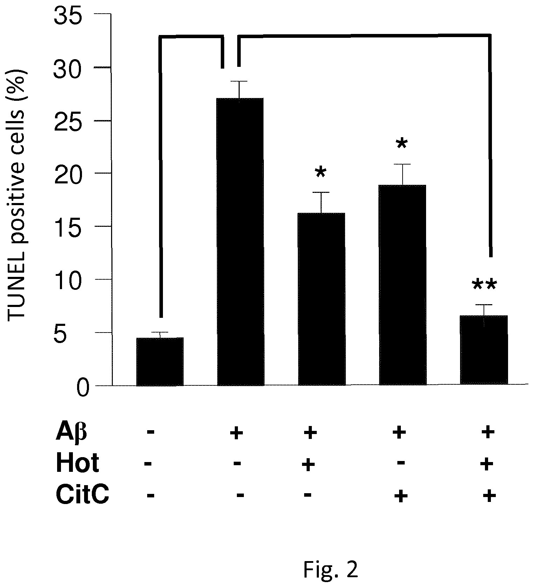

Figure 2 shows bar graphs showing the detection of apoptotic neuronal cells detected by TUNEL assay after treatment with homotaurine, citicoline and a combination thereof. The percentage of TUNEL positive cells was determined (n = 5). **, p,0.001; *, p,0.01 vs Ab25-35. The figure shows that the citicoline and homotaurine combination reduces mortality compared to the two individual substances.

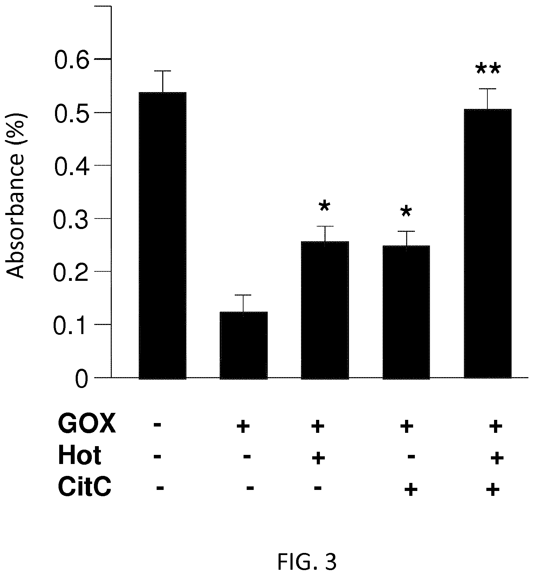

Figure 3 shows bar graphs showing the results on cell viability after treatment according to an embodiment of the invention, against the cytotoxicity induced by glucose oxidase (GOX) in pigmented human retina epithelial cells (n = 5). **, p,0.001; *, p,0.01 vs GOX.

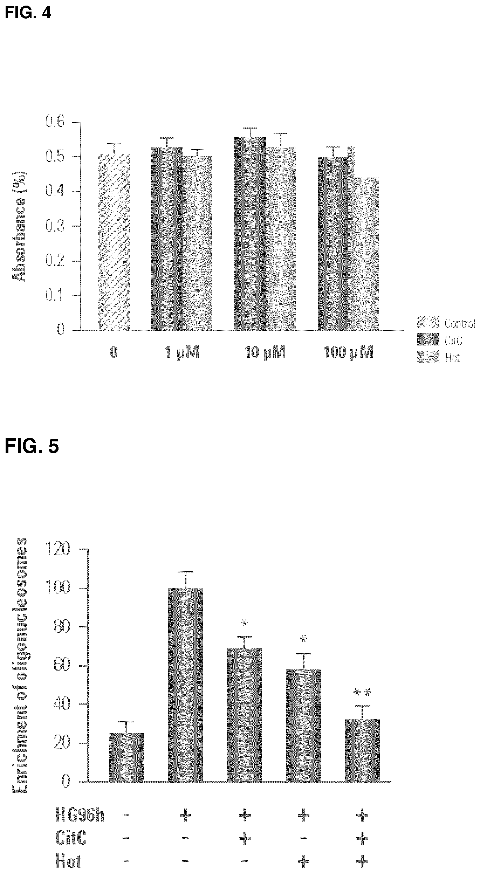

Figure 4 shows bar graphs showing the substantial absence of cytotoxicity of citicoline and homotaurine at different concentrations of 1, 10, 100 µM for 24h. Figure 5 shows bar graphs of comparative tests. Graphs illustrate the enrichment of oligonucleosomes in cells treated with High Glucose (HG) at 96 hours, compared with citicoline and homotaurine alone and in combination. The bar to the right, concerning the combined citicoline and homotaurine treatment, shows low apoptotic cell damage values and thus a protective effect.

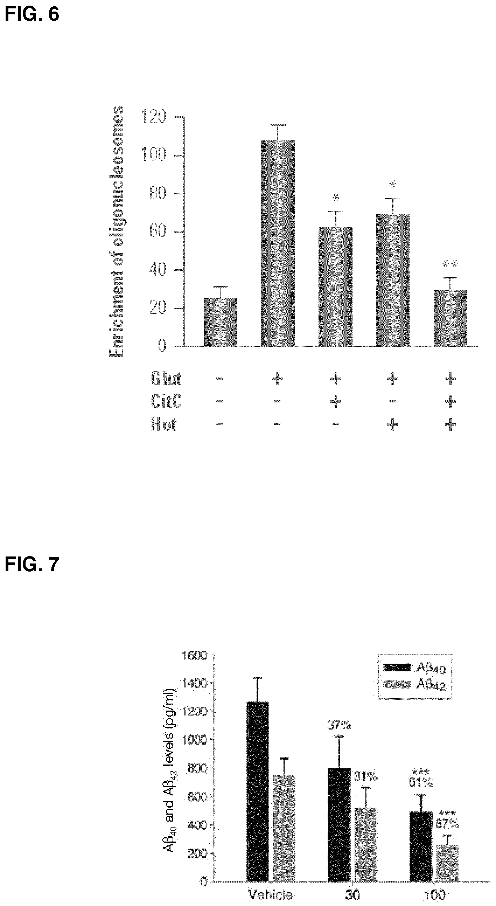

Figure 6 shows bar graphs concerning comparative tests that illustrate the enrichment of oligonucleosomes in cells treated with Glutamate at 96 hours, compared with citicoline and homotaurine alone and in combination.
Figure 7 shows bar graphs that show the ability of homotaurine to bind to peptides Aβ 40 and Aβ42 in blood plasma.

### DETAILED DESCRIPTION OF THE INVENTION

According to some aspects of the invention, inventors have discovered that the administration of a combination of citicoline and homotaurine as defined in the claims determines a synergistic effect of activation of certain neuronal receptors affected by certain central neurotransmitters that determine the regulation of the main cognitive functions.

This synergy of action allows obtaining a therapeutic or prophylactic treatment of neurodegenerative diseases and cognitive disorders that is greater than that found with the two active ingredients in the composition of the invention, taken alone.

It was found that taking the citicoline and homotaurine combination increases the biosynthesis of acetylcholine, causing an intense activation of certain important neuronal cortical functions, achieving a therapeutic effect with a reduced dose of active ingredients, thus reducing the occurrence of possible side effects, particularly those associated with the chronic treatment of brain involutional processes. According to one aspect thereof, the present invention therefore relates to a composition comprising a combination of citicoline or a pharmaceutically acceptable salt thereof and homotaurine and a physiologically acceptable carrier and/or excipient for use in the prevention or treatment of a neurodegenerative disease and/or a reversible cognitive disorder, a vascular cognitive impairment and/or a neurodegenerative ocular disease, wherein citicoline is in amount of 100 to 5000 mg and homotaurine is in amount of 5 to 500 mg.

As seen, an active ingredient component of the composition for use of the invention is citicoline.

Citicoline or 2-[{[5-(4-amino-2-oxopyrimidin-1-yl)-3,4-dihydroxyoxolan-2-yl]methoxy-hydroxyphosphoryl}oxy-hydroxyphosphoryl]oxyethyl-thrimethylazanium is an intermediate in the biosynthesis of structural phospholipids of neuronal cell membranes, in particular of phosphatidylcholine, which represents their main constituent.

Citicoline administered orally is rapidly absorbed and converted in the body into the choline and cytidine metabolites. Choline is an intermediate in the synthesis of acetylcholine, a neurotransmitter that plays an important role in the regulation of certain brain functions. Experimental evidence has demonstrated the ability of citicoline, administered orally, to determine a cholinergic activation in the central nervous system. This activity was surprisingly increased by administering citicoline combined with homotaurine, according to certain aspects of the invention. Cytidine, another metabolite of citicoline, is metabolized in the plasma into uridine, which at neuronal level is converted into cytidine triphosphate, a substance with nootropic activity.

The oral administration of citicoline or a salt thereof also increases the synthesis and levels of some catecholamines such as dopamine, norepinephrine and tyrosine in the cerebral cortex.

Moreover, citicoline has a high water solubility and bioavailability greater than 90%, features that make it suitable for oral administration, despite the prior art contemplates its use primarily through the parenteral route.

In experimental animal models, the applicant compared the bioavailability by oral and venous route of citicoline, labeling it with a radioactive label -[methyl-14C]. The percentage of radioactivity in plasma recorded over time showed an equivalence of the two alternative administration routes of citicoline of about 90%.

According to some embodiments, the citicoline in the composition for use of the present invention has a titer greater than 99.99%.

In some embodiments, citicoline is present in the form of a pharmaceutically acceptable salt such as citicoline sodium or potassium salt.

Typically, the citicoline used is highly pure and has an almost total absence of pollutants like allergens, heavy metals, additives and preservatives, for example each of these contaminants may be present in an amount of less than or equal to 0.001%.

According to some embodiments, the citicoline of the composition is obtained by a fermentation process.

In some embodiments, the composition for use of the invention contains citicoline in an amount of from 100 to 5000 mg, 250 to 1000 mg.

In some embodiments, the composition for use of the invention contains citicoline in an amount of from 0.1 to 95% by weight, 1 to 90% by weight, 10 to 85% by weight, 30 to 80% by weight.

There are numerous studies documenting how citicoline, typically taken individually, improves cognitive functions, regardless of whether the patients treated bear at least one apoE4 allele gene.

The following scientific publications are quoted as examples
Cotroneo AM, Castagna A, Putignano S, Lacava R, Fantò F, Monteleone F, Rocca F, Malara A, Gareri P. Effectiveness and safety of citicoline in mild vascular cognitive impairment: the IDEALE study. Clin Interv Aging. 2013;8:131-7*.*
Gruber SA, Sagar KA, Dahlgren MK, Gonenç A, Conn NA, Winer JP, Penetar D, Lukas SE. Citicoline Treatment Improves Measures of Impulsivity and Task Performance in Chronic Marijuana Smokers: A Pilot BOLD fMRI Study. Int J Neurol Neurother. 2015 Sep 30;2(3):1-8*.*
McGlade E, Agoston AM, DiMuzio J, Kizaki M, Nakazaki E, Kamiya T, Yurgelun-Todd D. The Effect of Citicoline Supplementation on Motor Speed and Attention in Adolescent Males. J Atten Disord. 2015 Jul 15. pii: 1087054715593633*.*
Knott V, de la Salle S, Smith D, Choueiry J, Impey D, Smith M, Beaudry E, Saghir S, Ilivitsky V, Labelle A. Effects of acute CDP-choline treatment on resting state brain oscillations in healthy volunteers. Neurosci Lett. 2015 Mar 30;591:121-5*.*
Alvarez-Sabín J, Ortega G, Jacas C, Santamarina E, Maisterra O, Ribo M, Molina C, Quintana M, Roman GC. Long-term treatment with citicoline may improve poststroke vascular cognitive impairment. Cerebrovasc Dis. 2013;35(2):146-54.
Zafonte RD, Bagiella E, Ansel BM, Novack TA, Friedewald WT, Hesdorffer DC, Timmons SD, Jallo J, Eisenberg H, Hart T, Ricker JH, Diaz-Arrastia R, Merchant RE, Temkin NR, Melton S, Dikmen SS. Effect of citicoline on functional and cognitive status among patients with traumatic brain injury: Citicoline Brain Injury Treatment Trial (COBRIT). JAMA. 2012 Nov 21;308(19):1993-2000*.*
Alvarez-Sabin J, Roman GC. Citicoline in vascular cognitive impairment and vascular dementia after stroke. Stroke. 2011 Jan;42(1 Suppl):S40-3*.*
*Garcia-Cobos R, Frank-Garcia A, Gutiérrez-Fernández M, Díez-Tejedor E.*
Citicoline, use in cognitive decline: vascular and degenerative. J Neurol Sci. 2010 Dec 15;299(1-2):188-92*.*

Another active component of the synergistic composition for use of the invention is homotaurine.

Homotaurine, 3-amino-1-propanesulfonic acid (3-APS) or tramiprosate (INN) is an organic compound similar to taurine. Homotaurine has the ability to bind to amyloidogenic proteins (β-amyioid protein), a group of proteins that are organized into extracellular fibrillar protein deposits from which the amyloid plaque in the brain structures originates. Once deposited in the brain, the amyloid protein forms senile plaques. Since the extracellular deposition of the β-amyioid protein in some brain regions and the presence of plaques is one of the typical signs of Alzheimer's disease, homotaurine, due to the specific property of inhibiting the formation of these plaques, has been proposed as a useful agent in the treatment of Alzheimer's disease.

Safety and pharmacological studies were conducted to verify the pharmacological action and toxicity of homotaurine on the central nervous system, on the gastrointestinal, respiratory and cardiovascular systems. By way of example, the following studies are mentioned as they were performed to evaluate the safety of use and tolerability and pharmacokinetic profile of homotaurine:
Greenberg SM, Rosand J, Schneider AT et al. A phase 2 study of tramiprosate for cerebral amyloid angiopathy. Alzheimer Dis Assoc Disord 2006; 20: 269-74*.*
Saumier D, Aisen PS, Gauthier S et al. Lessons learned in the use of volumetric MRI in therapeutic trials in Alzheimer's disease: the ALZHEMED (tramiprosate) experience. J Nutr Health Aging 2009; 13: 370-2*.*
Aisen PS, Gauthier S, Ferris SH et al. Tramiprosate in mild-tomoderate Alzheimer's disease - a randomized, double-blind, placebo-controlled, multi-centre study (the Alphase Study). Arch Med Sci 2011; 1: 102-11*.*
Saumier D, Duong A, Haine D, Garceau D, Sampalis J. Domainspecific cognitive effects of tramiprosate in patients with mild to moderate Alzheimer's disease: ADAS-cog subscale results from the Alphase Study. J Nutr Health Aging 2009; 13: 808-12*.*

In addition, a clinical study has demonstrated that the administration of 50 mg per day of homotaurine in individuals with an average decline of cognitive functions has a positive effect on atrophy of the hippocampus and episodic memory loss: Spalletta G, Cravello L, Gianni W, Piras F, et al. Homotaurine Effects on Hippocampal Volume Loss and Episodic Memory in Amnestic Mild Cognitive Impairment. J. Alzheimers Dis. 2016;50(3):807-16*.*

The attached Figure 7 modified by Gervais F, Paquette J, Morissette C, et al. Targeting Aβ peptide with tramiprosate for the treatment of brain amyloidosis. Neurobiol Aging 2007; 28: 537-47 shows how the treatment with homotaurine reduce plasma levels Aβ after an 8-week treatment with homotaurine with dosage of 30 or 100 mg/kg/day. The plasma levels were determined by ELISA. Specifically, the figure shows how homotaurine is able to reduce the values to Aβ40 and Aβ42 in plasma.

The applicant has now surprisingly found that when homotaurine is administered in conjunction with citicoline, a synergistic effect on the cholinergic transmission is obtained at the cortical level, which stimulates certain impaired brain functions in cognitive decline or dementia. This brain activity observed by the inventors is unexpected as it is different from that typical of homotaurine reported in the literature, which is based on inhibiting the β-amyioid plaque formation and deposition.

The inventors have also noted that the administration of an association of citicoline and homotaurine exerts a synergistic effect of stimulus on the cholinergic transmission that corrects those alterations of the central metabolism of acetylcholine which represent a leading cause of cognitive decline and Alzheimer's disease.

According to some aspects thereof, the present disclosure therefore relates to the use of citicoline and homotaurine in combination in the prevention and/or treatment of neurodegenerative diseases and cognitive disorders, as further defined in the claims. For these uses, the citicoline and homotaurine combination is conveniently formulated as a composition. In some embodiments, the composition for use of the invention contains homotaurine in an amount of from 5 to 500 mg, 20 to 200 mg.

For example, the composition for use of the invention may contain homotaurine in an amount of from 0.01% to 40% by weight, 0.5 to 20% by weight or 2 to 15% by weight.

The composition for use of the invention contains
- citicoline in an amount of from 100 to 5000 mg, homotaurine in an amount of from 5 to 500 mg;
- citicoline in an amount of from 250 to 1000 mg, homotaurine in an amount of from 20 to 200 mg.

Typically, homotaurine and citicoline, the two basic active ingredients of the composition of the invention, have a synergistic activity in preventing and/or treating neurodegenerative diseases and/or cognitive decline, including vascular cognitive impairment, senile dementia and/or the symptoms associated with these diseases. In the present application, the term "synergism or synergistic activity" means an activity that is greater than the sum of the activities of the individual active ingredient. In particular, the synergism occurs when at least two substances or active ingredients interact in a way that enhances or increases one or more of their effects. Therefore, two substances or active ingredients which produce apparently similar effects sometimes will produce exaggerated or reduced effects when used concurrently and a quantitative assessment is needed to distinguish these cases from a simple additive action (Tallarida RJ. Drug Synergism: its detection and applications. J PharmacolExpTher. 2001 Sep:298(3):865-72).

According to some embodiments, the composition of the invention further comprises a substance provided with a cellular antioxidant activity.

Within the scope of the present invention, the measurement of the antioxidant activity can be achieved with the "Oxygen-Radical Absorbance Capacity" (ORAC) method (Cao, G.; et al., (1993) Free Radic. Biol. Med. 14(3):303-11), or with the Total Radical-Trapping Antioxidant Parameter (TRAP) method (Ghiselli, A.; et al. (1995) Free Radic. Biol. Med. 18(1):29-36; Wayner, D. D et al. (1985) FEBS Lett. 187(1):33-7), which determine the ability of the substances to delay or block the ROS produced by generators of free radicals.

Within the scope of the invention, the term effective amount means the amount of homotaurine and citicoline which determines a desired prophylactic or therapeutic response, within the ranges defined in the claims.

Within the scope of the invention, a suitable substance provided with cellular antioxidant activity includes vitamin E and derivatives thereof.

Within the scope of the present invention, the term vitamin E and derivatives thereof is meant to include tocopherols (α, β, γ, δ), tocotrienols (α, β, γ, δ). A preferred tocopherol of the invention is α-tocopherol.

In some embodiments, the composition for use of the invention contains vitamin E, such as α-tocopherol, in an amount of from 0.01 to 300 mg, 0.1 to 50 mg, 1 to 30 mg.

In some embodiments, the composition for use of the invention contains vitamin E in an amount of from 0.001 to 20% by weight, 0.05 to 10% by weight or 0.5 to 5% by weight.

In some embodiments, the composition of the invention may comprise one or more additional substances or active ingredients.

In some embodiments, the composition for use of the invention further comprises one or more vitamins, such as vitamins B, niacin, vitamin A, vitamin C, vitamin PP, vitamins B being preferred.

According to some embodiments, the composition for use of the invention further comprises one or more micronutrients and/or minerals such as salts of Mg, K, Na, Zn, Fe, Cr, Se, Mn and others.

The term "carrier" as used herein indicates a medium, excipient, thinner with which the combination of therapeutic or active ingredients is administered.

Any carrier and/or excipient suitable for the desired preparation for administration to humans is contemplated for use with the compounds described in the present invention.

For purposes of the present application, the term "physiologically acceptable" is meant to indicate edible substances which are approved by health authorities for use in the pharmaceutical, nutritional, herbal or food applications.

A physiologically acceptable carrier may be a pharmaceutically acceptable carrier. In the scope of the present application, the term combination means that one or more active ingredients are added to or mixed with one or other ingredients.

The term combination or association should not therefore be construed in the meaning that the active ingredients are associated with each other with the formation of bonds of chemical type or other type.

The compositions for use of the present invention comprise any composition made by administering the association of active ingredients of the present invention and a physiologically or pharmaceutically acceptable carrier. Such compositions are suitable for food, dietary, pharmaceutical or nutritional use in mammals, in particular humans.

The composition for use of the invention can take a variety of forms of preparation, depending on the desired route of administration.

The composition for use of the invention may be in solid form.

When the composition for use of the invention is in solid form, it can be in the form of a tablet, capsule, powder, granules.

When the composition is in liquid form, it can be in the form of a suspension, emulsion, solution. In these cases, the carrier is liquid and can be selected for example from water, glycol, oils, alcohols and mixtures thereof. According to some embodiments, the composition can be in liquid form of eye drops or semi-fluid, such as eye gel to be instilled in the eye. In these embodiments for ophthalic use, the composition typically is a water-based solution or suspension which may contain one or more of preservatives such as benzalkonium chloride, surfactants, boric acid, sodium chloride, buffering agents such as anhydrous sodium sulfate and/or other additives, such as xanthan gum commonly used for the preparation of ophthalmic eye drops or gels.

Typically, the compositions for use of the invention are in solid form, such as in tablet form.

The preparations in solid form may include one or more carriers such as starches, sugars, microcrystalline cellulose, and optionally diluents, granulating agents, lubricants, binders, disintegrating agents.

Typically, tablets, pills, capsules, granules may also contain a binder such as acacia, tragacanth, cornstarch or gelatin; excipients such as calcium phosphate; a disintegrating agent such as cornstarch, potato starch, alginic acid; a lubricant such as magnesium stearate; a sweetening agent such as sucrose, lactose or saccharin. If desired, the tablets may be coated using traditional techniques.

When the pharmaceutical unit form is a capsule, it may contain a liquid carrier such as a fat oil in addition to the above materials.

According to certain embodiments, the composition for use of the invention contains a cellulose-based excipient which comprises i) cellulose organic esters, for example selected from cellulose acetate, cellulose proprionate, cellulose triacetate, cellulose acetate proprionate, cellulose acetate butyrate, ii) cellulose inorganic esters, for example selected from nitrocellulose, cellulose sulfate, iii) cellulose ethers selected from a) cellulose alkyl ethers, for example selected from methylcellulose, ethylcellulose, ethylmethylcellulose; b) cellulose hydroxyalkylethers, for example selected from hydroxyethyl cellulose, hydroxypropyl cellulose, hydroxypropyl cellulose, hydroxyethyl cellulose, hydroxypropyl methylcellulose, ethylhydroxyethyl cellulose; c) carboxyalkyl cellulose such as carboxymethylcellulose, salts and mixtures thereof. In certain embodiments, the cellulose-based excipients are crosslinked with physiologically acceptable crosslinking agents.

The composition may also include flavoring agents, preservatives, dyeing agents and the like.

In certain embodiments, the composition for use of the invention is in solid form of a tablet. According to preferred embodiments, the composition of the invention is in the form of gastro-resistant tablet.

According to certain embodiments, the composition is in a form for the modulated or controlled release of the active ingredients. By way of example, the composition may be a retard, rapid-release or slow-fast release formulation. Typically, modulated or controlled release formulations contain one or more of cellulose-based excipients, in particular of the type described above.

In certain embodiments, the composition for use of the invention comprises a hydrogenated fatty acid as an excipient, preferably having a chain with 3 to 20 carbon atoms, 14 to 18 carbon atoms. A typical example of a suitable hydrogenated fatty acid is hydrogenated palm oil.

In some embodiments, the active ingredients contained in the composition for use of the present invention can be combined or mixed as active ingredients in intimate admixture with a suitable edible carrier and/or an excipient according to the traditional pharmaceutical and food or nutritional industry techniques.

The compositions for pharmaceutical or nutritional use can be appropriately presented in a single pharmaceutical form and prepared by any of the methods well known in the pharmaceutical or food technique.

The amount of active compound in the compositions is defined by the claims.

In some embodiments, the composition for use of the invention further comprises one or more additional components such as additives, fillers, stabilizers, emulsifiers, textured, film formers, plasticizers, wetting agents and thickeners.

Various other materials may be present as coatings or to modify the physical form of the pharmaceutical unit. For example, the tablets may be coated with shellac, sugar or both. In order to prevent the disintegration during transit through the upper gastrointestinal tract, the composition may be a gastro-resistant or enteric-coated formulation.

In certain embodiments in which the formulation is in liquid form, for example in cases of a syrup or elixir, it may contain in addition to the association of active ingredients, sucrose as a sweetening agent, methyl and propylparaben as preservatives, a coloring agent and a flavoring agent such as cherry or orange flavor.

In some embodiments, the formulation will contain amounts of active ingredients that depend on the severity of the cognitive decline form and of the related symptomatology, of the condition, the additional current therapies, the individual health condition and the response to the association of the active ingredients, within the scope of the claims.

According to some embodiments, the composition for use of the invention is a drug. According to some embodiments, the composition for use of the invention is a nutraceutical, food supplement, a nutritional or dietary product.

The present disclosure also relates to a composition comprising a combination of citicoline and homotaurine and a physiologically acceptable carrier and/or excipient for use in the prevention or treatment of neurodegenerative diseases and cognitive disorders, as defined in the claims.

The composition for use according to certain aspects of the invention comprise the treatment of neurodegenerative disorders, forms of cognitive decline such as dementia, memory loss, senile dementia, forms of cognitive decline of vascular origin, protection from neuronal damage, for example as a result of ischemic events, as far as encompassed by the claims.

The administration of the composition is particularly indicated for use in the case of alterations at the central level (SNC) of the acetylcholine metabolism. In these cases, the administration of the composition results in an improvement of cognitive skills, in particular of memory and reaction times.

Studies to evaluate the pharmacokinetic-pharmacodynamic effect of compositions for use of the invention have provided positive indications about the activity. In particular, in a randomized double-blind, placebo-controlled trial in which 58 subjects with from medium to moderate Alzheimer's disease were administered 50, 100, 150 mg of homotaurine in combination with 500, 750 and 1000 mg of sodium citicoline. In the trial it was found that the two active ingredients at these concentrations have crossed the blood-brain barrier and the lowest dose (50 mg homotaurine and 500 citicoline) has reduced the symptomatology and reduced β amyloid proteins in the spinal fluid of patients treated.

It was also observed that the citicoline and homocysteine combination has the following effects:
- It increases the synthesis of phospholipids, in particular phosphatidylcholine and sphingomyelin, stabilizing cell membranes and determining a protective and reparative effect on the same;
- It inhibits the activation of phospholipase A2 by suppressing the release of free fatty acids, including arachidonic acid, accelerating the resorption of cerebral edema and the progression of ischemic damage;
- It inhibits apoptopic mechanisms associated with brain ischemia, in particular it inhibits the release of glutamate during ischemia;
- It increases neuroplasticity by preventing or reducing any neurological damage in case of hypoxia or ischemia;
- It improves attention, spatial-temporal orientation in post-stroke patients or those with vascular cognitive decline.

According to some aspects, the composition for use of the invention containing homotaurine and citicoline according to any one of the embodiments described above finds application in the ophthalmological field, specifically in the prevention and/or treatment of certain degenerative eye diseases such as dry or exudative macular degeneration or AMD. The composition for use of the invention containing homotaurine and citicoline according to any one of the embodiments described above is also applied in the treatment of glaucoma. The neuro-protective features of the composition for use of the invention contribute to this therapeutic activity.

The following examples illustrate the present invention. The scope of protections is clear from the appended claims

### EXAMPLE 1

Composition in the form of gastro-resistant tablets for the prevention and treatment of cognitive decline having the following formulation:

| | |
|---|---|
| Base citicoline | 500 mg |
| Homotaurine | 50 mg |
| Vitamin E (α TEq) | 12 mg |

The daily dose for prevention or treatment is 1 tablet daily.

### EXAMPLE 2

Composition in tablet form for the treatment of vascular cognitive decline having the following formulation

| | |
|---|---|
| Base citicoline | 800 mg |
| Homotaurine | 100 mg |

### Coating:

| | |
|---|---|
| Ethyl cellulose | 2.2 g |
| Talc | 0.2 g |
| Carnauba wax | 0.1 g |
| Total equal to | 2.5 g |

### EXAMPLE 3

### Cell culture of neurons

Neurons of rat hippocampus (H 19-7) and human retina pigment epithelium (ARPE-19) were purchased from American Type Culture Collection (Manassas, VA, USA) and grown according to the manufacturer's instructions.

### MTT assay of neurons and cells of the retina

The MTT assay was used to measure the viability of both neuronal and retina cells. The cells were prepared in a 96-well plate in DMEM-F12 with 7% FBS. After cultured in DMEM-F12 without serum for 12 h, Ab25-35 (20 mM) or glucose oxidase (GOX) alone or in combination with homotaurine (Hom), citicoline (CITC) or Hom + CITC were added to the medium. Twenty-four hours later, the cells were incubated with 10 mL MTT (Roche, 0.5 mg/ml) at 37 °C for 4 hours, then the crystals were dissolved in 150 mL DMSO. Absorbance at 570 nm was measured with a spectrophotometer (TECAN Infinite M200, Switzerland).

### dUTP Nick End Labeling staining (TUNEL) mediated by TdT- Cell apoptosis in neurons

For the analysis of apoptosis, the fragmentation of neuronal cell DNA was measured with a in situ cell death detection kit (Roche Diagnostics Corporation, Indianapolis, IN, USA) according to the manufacturer's instructions. Following the TUNEL staining, the cell nuclei were stained with DAPI contrast dye (Sigma-Aldrich, St Louis, MO, USA). The neuronal cells positive to the TUNEL staining were counted under a fluorescence microscope (Nikon 80i, Japan), in 5 fields (2006 magnification) randomly selected, and the average speed of apoptotic cells was calculated.

### The combination of Hom and CITC improved the cellular viability of neuronal cells treated with Ab

Concentrations of 20 µM Ab25-35 can induce evident cytotoxicity in neuronal cells, as indicated by the MTT assay. The respective concentrations of Hom and CITC were selected optimally at 1 µM for subsequent evaluation of their potential synergistic effect against neuronal damage induced by Ab25-35. The results of the colorimetric TUNEL assay further validated a protective synergistic effect between Hom and CITC against cellular apoptosis mediated by Ab25-35.

### The combination of Hom and CITC improved the cellular viability of retina pigmented epithelial cells treated with GOX

Glucose oxidase (GOX) 50 mU/ml, which generates hydrogen peroxide in the culture medium, induces cytotoxicity in cells of the retina. Both Hom and CITC 1 µM were able to improve the cell viability. The combination of the two compounds exhibits the (maximum) synergistic cytoprotective effect.

### Concentrations used for the two compounds

As shown in Figure 4, the cytotoxicity of citicoline and homotaurine on the cell cultures was evaluated at different concentrations (1, 10, 100 µM) for 24 h. None of the concentrations induced cell death. In addition, the MTT assay shows that the retinal cells are well preserved in the cultures treated with citicoline (CITC) or homotaurine (Hot), with no evidence of selective toxicity at 1, 10 or 100 µM.

### EXAMPLE 4

Experimental evidence of synergism citicoline and homotaurine.

The synergistic activity of the citicoline and homotaurine combination was further evaluated in terms of detection of apoptosis by an assay other than TUNEL. Specifically, the levels of apoptosis were analyzed by immunodetection of oligonucleosomes released by the nucleus to the cytoplasm of apoptotic neurons. The sandwich enzyme-linked immunosorbent assay (ELISA) was used. To induce apoptotic cell injury and to evaluate the synergistic and neuroprotective activity of the two compounds, the cell cultures were treated with high concentrations of glucose (30 mM) for 96 hours and glutamate (100 µM) for 25 minutes.

As shown in Figure 5, treatment with a formulation containing citicoline and homotaurine has reduced the rate of apoptosis in cells treated with High Glucose (HG) showing a synergistic effect. The cell apoptosis was determined by sandwich enzyme-linked immunosorbent assay.

Specifically, the enrichment of oligonucleosomes was evaluated. *, P <0.001 vs High Glucose 96 hours (HG96h). **, p<0.001 vs CitC and Hot alone.

Moreover, the data of Figure 6 show how the treatment with a formulation containing citicoline and homotaurine has reduced the rate of apoptosis in cells treated with Glutamate (Glut) showing a synergistic effect.

The cell apoptosis was determined by sandwich enzyme-linked immunosorbent assay. The enrichment of oligonucleosomi was evaluated. *, P <0.001 vs Glutamate (Glut). **, p<0.001 vs CitC and Hot alone.

## Claims

1. A composition comprising a combination of citicoline or a pharmaceutically acceptable salt thereof and homotaurine and a physiologically acceptable carrier and/or excipient for use in the prevention or treatment of a neurodegenerative disease and/or a reversible cognitive disorder, a vascular cognitive impairment and/or a neurodegenerative ocular disease, wherein citicoline is present in an amount of 100 to 5000 mg and homotaurine is present in an amount of 5 to 500 mg.

2. A composition for use according to claim 1, further comprising a substance provided with a cellular antioxidant activity selected from tocopherols α, β, γ or δ, tocotrienols α, β, γ or δ.

3. A composition for use according to claim 2, wherein said tocopherol is α-tocopherol or vitamin E.

4. A composition for use according to any one of claims 1 to 3, **characterized in that** it is in the form for oral administration.

5. A composition for use according to any one of claims 1 to 4, **characterized in that** it is a dietary supplement, a drug or a food supplement.

6. A composition for use according to any one of claims 1-5, **characterized in that** it is in the form of a tablet.

7. A composition for use according to claim 6, wherein said tablet is gastro-resistant.

8. A composition for use according to claim 1, wherein said cognitive disorder is senile dementia, Alzheimer's disease, amnestic deficits, and wherein said neurodegenerative ocular disease is glaucoma or macular degeneration.

## Patentansprüche

1. Zusammensetzung, umfassend eine Kombination von Citicolin oder einem pharmazeutisch akzeptablen Salz desselben und Homotaurin und einem physiologisch akzeptablen Trägerstoff und/oder Hilfsstoff zur Verwendung in der Vorbeugung oder Behandlung einer neurodegenerativen Krankheit und/oder einer reversiblen kognitiven Störung, einer vaskulären kognitiven Beeinträchtigung und/oder einer neurodegenerativen Augenkrankheit, wobei Citicolin in einer Menge von 100 bis 5000 mg vorhanden ist und Homotaurin in einer Menge von 5 bis 500 mg vorhanden ist.

2. Zusammensetzung zur Verwendung nach Anspruch 1, welche zudem einen mit einer zellulären antioxidativen Aktivität ausgestatteten Stoff umfasst, der aus Tocopherolen α, β, γ oder δ, Tocotrienolen α, β, γ oder δ ausgewählt ist.

3. Zusammensetzung zur Verwendung nach Anspruch 2, wobei das Tocopherol α-Tocopherol oder Vitamin E ist.

4. Zusammensetzung zur Verwendung nach einem der Ansprüche 1 bis 3, **dadurch gekennzeichnet, dass** sie in der Form für orale Verabreichung ist.

5. Zusammensetzung zur Verwendung nach einem der Ansprüche 1 bis 4, **dadurch gekennzeichnet, dass** sie eine diätetische Ergänzung, ein Arzneimittel oder eine Nahrungsergänzung ist.

6. Zusammensetzung zur Verwendung nach einem der Ansprüche 1 bis 5, **dadurch gekennzeichnet, dass** sie in der Form einer Tablette ist.

7. Zusammensetzung zur Verwendung nach Anspruch 6, wobei die Tablette magensaftresistent ist.

8. Zusammensetzung zur Verwendung nach Anspruch 1, wobei die kognitive Störung senile Demenz, Alzheimerkrankheit oder Gedächtnisdefizite ist, und wobei die neurodegenerative Augenkrankheit Glaukom oder Makuladegeneration ist.

## Revendications

1. Composition comprenant une combinaison de citicoline ou d'un sel pharmaceutiquement acceptable de celle-ci et d'homotaurine et un support et/ou excipient physiologiquement acceptable à utiliser dans la prévention ou le traitement d'une maladie neuro-dégénérative et/ou d'un trouble cognitif réversible, d'une déficience cognitive vasculaire et/ou d'une maladie oculaire neuro-dégénérative, dans laquelle la citicoline est présente en une quantité de 100 à 5 000 mg et l'homotaurine est présente en une quantité de 5 à 500 mg.

2. Composition à utiliser selon la revendication 1, comprenant en outre une substance dotée d'une activité antioxydante cellulaire choisie parmi les tocophérols α, β, γ ou δ, les tocotriénols α, β, γ ou δ.

3. Composition à utiliser selon la revendication 2, dans laquelle ledit tocophérol est un tocophérol ou une vitamine E.

4. Composition à utiliser selon l'une quelconque des revendications 1 à 3, **caractérisée en ce qu'**elle se présente sous la forme d'une administration orale.

5. Composition à utiliser selon l'une quelconque des revendications 1 à 4, **caractérisée en ce qu'**elle est un supplément diététique, un médicament ou un complément alimentaire.

6. Composition à utiliser selon l'une quelconque des revendications 1 à 5, **caractérisée en ce qu'**elle se présente sous la forme d'un comprimé.

7. Composition à utiliser selon la revendication 6, dans laquelle ledit comprimé est gastro-résistant.

8. Composition à utiliser selon la revendication 1, dans laquelle ledit trouble cognitif est la démence sénile, la maladie d'Alzheimer, les déficits mnésiques, et dans laquelle ladite maladie oculaire neuro-dégénérative est le glaucome ou la dégénérescence maculaire.
